# EUROPEAN PATENT SPECIFICATION

(11) **EP 1 408 823 B1**
(45) Date of publication and mention of the grant of the patent: **12.05.2010**
(21) Application number: 02748235.5
(22) Date of filing: 23.07.2002
(51) Int. Cl.: A61B 5/00, A61B 5/0402

(54) **MOBILE PATIENT MONITOR**
MOBILER PATIENTENMONITOR
DISPOSITIF DE SURVEILLANCE POUR UN PATIENT MOBILE

(30) Priority: 26.07.2001 US 308070 P
(43) Date of publication of application: 21.04.2004
(73) Proprietor: DATASCOPE INVESTMENT CORP., Montvale, N.J. 07645 (US)
(72) Inventor: SAPER, Lawrence, Montvale, NJ 07645 (US); KINAST, Eric, Santa Ana, CA 92706 (US)
(74) Representative: Bergstrand, Mikael Gudmundsson
(86) International application number: PCT/US2002/023434
(87) International publication number: WO 2003/009749

(56) References cited:
- EP-A- 0 602 459
- WO-A-00/62665
- WO-A-97/00708
- WO-A-98/39749
- US-A- 5 417 222
- US-A- 6 090 056
- US-A- 6 093 146
- US-B1- 6 289 238
- US-B1- 6 405 083
- US-B1- 6 440 067

## Description

### BACKGROUND OF THE INVENTION

### Field of the Invention:

The invention relates to a medical monitoring system. More particularly, the invention relates to a system for monitoring the condition and location of a subject or patient.

### Description of the Prior Art:

The prior art is replete with various systems to monitor hospitalized patients and provide patient data to a central location. One type of system, employed in the intensive care units (ICU) of hospitals where vital signs of a patient are monitored, is a bedside system. In such a system the patient is confined to a bed and is suitably connected to sensors so as to allow for physiological medical information to be transmitted to a central location via cables or other means.

More modern systems generally equip a patient with a transmitter and receiver and utilize wireless transmission, such as telemetry systems, to communicate the patient's physiological medical information. Sensors placed on the patient monitor electrical signals produced by the patient to provide, for example, electrocardiogram (EKG) signals. These subject or physiological signals are then transmitted by antennas, conventional radio links or by other radio frequency (RF) techniques. Existing ambulatory systems can provide various signals relating to the monitoring, for example, the patient's temperature, heart rate and so on. Essentially, the type of signal which can be transmitted by such systems includes any type of signal which can be measured by conventional sensors which are applied to the skin or otherwise implanted in the patient.

Prior art monitoring systems also exist which give an indication of the location of the patient. A typical prior art system is described in U.S. Pat. No. 4,958,645 entitled MultiChannel Digital Medical Telemetry System, which issued on Sep. 25, 1990 to Cadell et al. The medical radio telemetry system described therein utilizes a plurality of antennas, which are distributed throughout a hospital or other premises. The patient is outfitted with a radio receiver and transmitter to collect a patient physiological signal, including, for example, the patient's temperature, heart rate, pacer rate, respiration rate, brain activity level and blood pressure level. The transmitter and receiver associated with the patient operate in conjunction with one or more room locator transmitters spaced in rooms where the patient is being monitored. The room locator transmitters emit signals indicative of the room they are emanating from. Signals from the room locator transmitters are combined with the patient signals so as to enable hospital staff to monitor the location of patients.

U.S. Pat. No. 4,981,141, entitled Wireless Electrocardiogram Monitoring System, issued on Jan. 1, 1991 to Segalowitz, discloses an electrocardiographic monitoring system where the patient's heart-signaling sensing electrodes are each coupled to the heart-signal monitor/recorder by respective wireless transmitters and corresponding respective wireless receivers in a base unit. Each transmitter/receiver combination operates at a separate radio frequency to provide a zero or reference signal at a base unit and which is used to modulate a signal transmitter at the base unit. Each modulated signal, when received and demodulated provides information concerning signal sensed by a respective electrode carried by the patient, as for example, the right-leg electrode, etc.

It is clear from the above that it is extremely desirable to monitor various vital signs of an ambulatory patient. This is even more important due to the recent trend to get patients ambulating as soon as possible. It is also important to determine the location of a monitored patient within, for example, the confines of a hospital or other area so as to ensure expedient care in the case of an emergency, for example.

The disadvantage to the prior art bedside system is clear: the system does not allow for patient mobilization. The more modern telemetry monitoring systems, although superior to the bedside systems, also have a number of significant drawbacks.

One-way communication systems are designed such that they use a different channel for each patient in order to avoid interference between patient signals, i.e. patient signals arriving at a receiver at the same. When the number of patients becomes large these systems become expensive, complex and unwieldy, requiring many channels and complex receivers. Often, in order to avoid this problem, as well as to assist in patient location, as in U.S. Patent No. 4,958,645, designers revert to a two-way communication system. However, as detailed below, two-communication adds considerable complexity to the system and precludes a patient-worn device from being small, light, and low-power. Document EP 0602459 discloses a device as described in the preambule of claim 1.

The more modern telemetry systems often employ two-way communications for system management purposes to better coordinate the actions of the individual patient transmitters, such that patient signal transmissions are scheduled in an orderly and efficient manner to avoid interference and make full use of the available bandwidth. Each transmitter is assigned a time slot, during which it has exclusive use of the channel to transmit a burst of data. In order to keep each transmitter operating in its assigned time slot, the transmitters must be coordinated via, for example, a transmitter broadcasted beacon, thus rendering the system two-way; the first link for transmission of the patient signal to the transmitter and the second link for transmission of the coordinating beacon back to the patient. Because the time slots can be precisely assigned, this two-communication system makes very efficient use of the bandwidth, but at the expense of requiring a two-way link.

The two-way communication capability, which requires a receiver as well as a transmitter, adds considerable complexity to the system and precludes a patient-worn device from being small, light, and low-power. If the need to manage the transmitters can be avoided, then one-way communications can be used and the monitor could be small, light and use low power.

### SUMMARY OF THE INVENTION

Accordingly, it is an object of the invention to produce a one-way communication, small, light and low power telemetry system for monitoring the health and location of a subject or patient.

Currently, unmonitored patients may succumb to sudden cardiac arrest without knowledge of hospital staff. The present system attempts to correct this problem by providing continuous vigilance over those patients that might not otherwise be monitored.

The device comprises one or more transmission means for acquiring and analyzing a subject signal and for transmitting data resulting from the analysis to one or more receiving means. Said receiving means receive the data from the transmission means and communicate the data to a central station means. Said central station means receives and analyzes the data from the receiving means, optionally including data related to the location of the patient, and notifies a user, i.e. hospital staff, of an alert or other predetermined patient condition.

The device size and power consumption are minimized by (i) analyzing the patient signal at the transmission means end, the patient side of the system, and only transmitting vital sign or other important physiological patient information to the central station and (ii) limiting the range of transmission means. Limiting the range of the transmission means also facilitates patient location. Given the shortened range only a few patients should be within range of any given receiving means at any one time.

Note that the present invention is not limited to a hospital setting to monitor patients. Rather, the present invention may be employed whenever there is a need to track the condition and/or location of a subject, which may include inanimate objects, animal or humans, in a defined area.

To the accomplishment of the above and related objects the invention may be embodied in the form illustrated in the accompanying drawings. Attention is called to the fact, however, that the drawings are illustrative only. Variations are contemplated as being part of the invention, limited only by the scope of the claims.

### BRIEF DESCRIPTION OF THE DRAWINGS

In the drawings, like elements are depicted by like reference numerals. The drawings are briefly described as follows.

Figure 1 is a block diagram of the mobile monitoring system of the present invention, showing the relationship of several patients to multiple receiving means.

Figure 2 is a perspective view of the patient transmission means.

Figure 3 is an electronic block diagram of the patient transmission means of Figure 2.

### DETAILED DESCRIPTION OF THE PREFERRED EMBODIMENTS

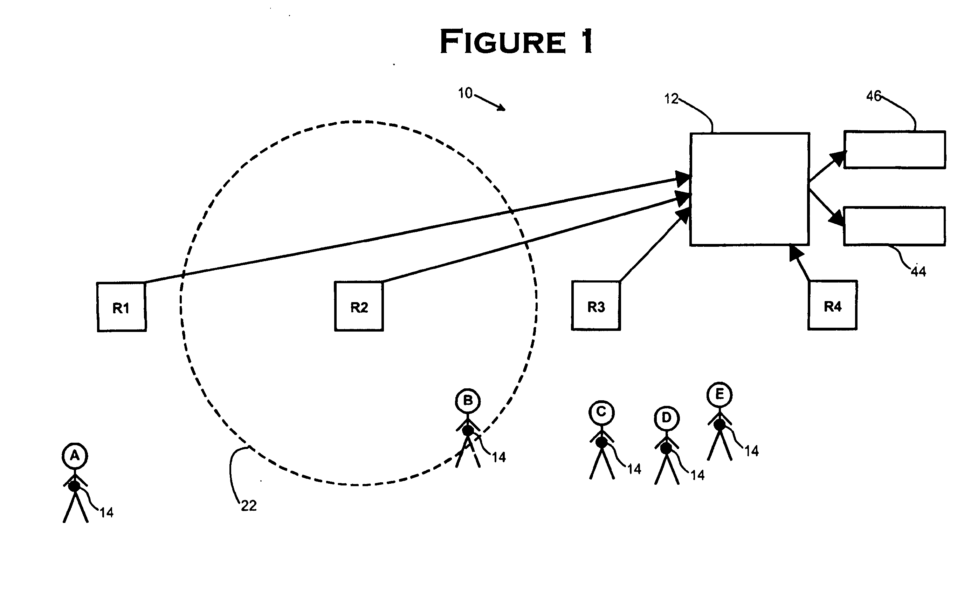

An overview of the preferred embodiment of the system 10 is shown in Figure 1. System 10 consists of a central station means 12 linked to a series of receiving means, labeled R1-R4, distributed throughout a defined area, such as the patient areas of a hospital. Each of five patients, labeled A-E, have an associated transmission means 14 for acquiring his or her physiologic signal, processing it to obtain medical condition data and then transmitting this medical condition data to one or more of the receiving means, R1-R4, for transmittal to central station means 12. In the present example the physiologic signal acquired is the patient's ECG waveform and the medical condition data consists of heart rate and rhythm.

Note that transmission means 14 will vary depending on the type of subject and range of conditions being monitored. The present invention is not limited to monitoring cardiac health of a patient in a hospital setting. One may want to track the movements of a turtle in a defined area, for example, and monitor the water content of its shell.

In the preferred embodiment, however, transmission means 14 is adhered to a patient's chest and comprises a device capable of measuring a patient's physiological signal, preferably the ECG signal, and analyzing this signal for patient medical condition data, such as heart rate and rhythm. More specifically, one transmission means 14 is connected to each patient, A through E.

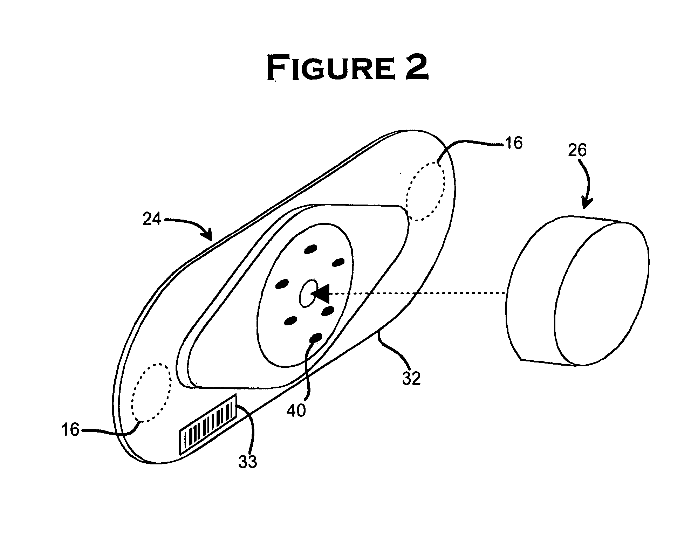

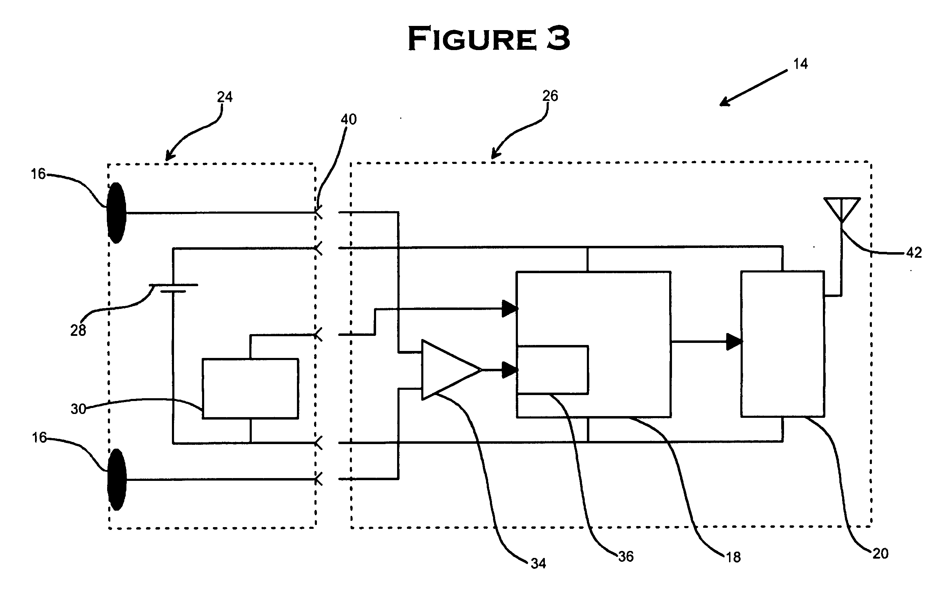

In general, transmission means 14 comprises a signal sensing means 16, a computing means 18, and a transmitter 20 (see Figures 2 and 3). Signal sensing means 16 may comprise one or more electrodes (as illustrated in Figures 2 and 3), a plethysmograph sensor, or other heart beat detection means. Computing means 18 analyzes a subject signal and produces patient condition data based on the subject signal. Computing means 18 may comprise a microprocessor, microcontroller, ASIC, or other programmable logic. Transmitter 20 transmits the patient condition data to central station means 12. Transmitter 20 may comprise a radio frequency, infrared, or ultrasonic device, or other device know in the art capable of transmitting bursts of data. The preferred embodiment of transmission means 14 is detailed below.

Receiver means R1-R4 comprise any device known in the art capable of receiving bursts or a stream of data and communicating this data to central station means 12. In this example, receiver means R1-R4 comprise a radio frequency receiver, means for communicating with central station means 12, and computing means(all not shown). Computing means may comprise a microprocessor, microcontroller, ASIC, or other programmable logic. Means for communicating with central station means 12 may comprise any of various computer data network communication devices, such as a wireless network, or wired network having either star, multidrop, or ring topology.

Central station means 12 may comprise any device capable of receiving and analyzing bursts or streams of data. Central station means 12 monitors each patient's medical condition data for predetermined rate and rhythm alarms. When an alarm condition is detected an alert is sounded or displayed on an associated display (not shown). Central station means 12 may also be configured to directly summon a response team, by means such as an interface to a telephone or pager system. By identifying the particular receiving means, R1-R4, picking up a given patient's signal the patient's location is known. Central station means 12 may comprise custom software running on a PC, equipped with suitable commercial wired or wireless network connectivity, or other computing platform.

Note that despite the system 10's optimization to work for short bursts of data, as opposed to extended or continuous signals, due to interference concerns, central station means 12 may receive and analyze physiological signals.

As indicated above, Figure 1 illustrates a schematic representation of system 10 of the present invention. Five patients, labeled A through E, are being monitored by four receiving means, designated R1 through R4. Note that the number of patients and receiving means may vary and that the numbers used in this example are for illustration purposes only. Each receiving means has a coverage radius overlapping that of at least an adjacent receiving means, as is illustrated by the dotted circle, labeled 22, surrounding receiving means R2. In the example illustrated in Figure 1, patient A is in range of only receiving means, namely, R1. However, patient B is in range of both receiving means R2 and R3. Further, receiving means R3 is also receiving signals from patients C, D, and E. Patient E is also in range of receiving means R4. As a patient moves, his signal will pass out of range of his original receiving means, and into range of an adjacent receiving means, with some overlap.

System 10 of the present invention is designed so as to handle patients within range of two or more receiving means at one point and designed such that patient condition data from multiple patients arriving at a single receiving means will not interference with each other. The system is also designed such that there is no loss of data or manual intervention as a patient moves from one receiving means to the next. Furthermore, transmission means 14 includes in the patient condition data information relating to the patient identity, since it is not known in advance where each patient's signal will be received.

Some known telemetry systems, as heretofore discussed, require bidirectional communication to manage the communication process itself. For example, in some known systems a central point sends a polling request to interrogate each remote unit, which then responds with data, as a means of allowing multiple devices to share a single channel. In order to minimize power and complexity, the present system uses unidirectional communications only, but at the expense of loosing some ability to manage the communication process.

The inventor of the present invention has successfully designed a mobile patient monitoring system 10, employing a uni-directional system, with minimal interference issues. Interference is minimized by transmitting to central station means 12 patient condition data, i.e. vital signs, rather than the patient's physiological signal, i.e. ECG waveform. Information is transmitted in short bursts, at low duty cycle, to minimize interference, i.e. the chance that information from different patients arrive at a single receiving means at the same time. In contrast to ECG waveforms, the amount of data associated with vital signs is small and changes slowly, and thus, is amenable to short burst transmissions. Further, given that vital signs, such as heart rate and rhythm, are based on averaging information from several heart beats, and thus do not change instantaneously, it is adequate to update the data every second or two. This is in contrast to ECG waveforms which need to be updated much more frequently to maintain a smooth waveform. Note that occasional interference of patient data does not significantly affect the monitoring value of the present system because data updating is quite redundant; that is, the loss of an occasional update does not severely limit the value of the data, and adequate information can be obtained by simply waiting for the next update. Given this, it is not necessary to request that the lost data be retransmitted.

In accordance with the above, present system 10 uses multiple transmission means 14, which use the same channel but without any coordination, i.e. they are variable in timing and asynchronous. In other words, one patient transmission means transmits bursts of data to receiving means without any attempt to make sure that another transmission means is not transmitting at the same time. While this may result in occasional interference between competing transmission means 14, and therefore occasional loss of data, if the interference is made sufficiently rare it is not objectionable.

Each cycle has an active phase in which energy modulated with the patient condition data is emitted and a longer inactive phase in which no energy is emitted. To avoid overlap of the active periods, the duration of the active phase should be less, and preferably much less, than the period of a complete cycle divided by a predetermined maximum number of transmission means anticipated to be within range of any one receiving point at any one time. The shorter the active phase is made, the less the probability of overlaps and associated interference.

As indicated above, in order to minimize interference, transmission means 14 sends updates as short bursts of data, preferably lasting approximately 5 milliseconds with burst of data at intervals averaging one second, with a random variation. Thus, transmitter 20 is in an active phase only about 0.5% of the time. If two such transmission means, without any synchronization between them, are sharing a single receiving means, there is a small probability that they will occasionally interfere, with the result that they both loose one update. However, because of the randomness of the time to the next update, it is highly unlikely that they will lose the next update as well. In the very unlikely event that this happens, there is an even smaller, essentially negligible, probability that this would happen a third time. Therefore, the worst that could happen as a result of the mutual interference of these unmanaged transmitters 20 is a rare delay in the data update by a second or two at most. As more similar transmission means are brought into range of this one receiving means, the probability of interference increases, but since the patient transmission means is designed with a short range, it limits the number of transmission means 14 that can be within range of each receiving means, R1-R4, while at the same time reducing power consumption.

Transmission means 14 operate on a duty cycle which is determined by taking into consideration and optimizing the following variables: (a) the amount of patient condition data that must be transmitted; (b) the speed of the transmission; (c) how often the patient condition data must be sent; (d) the allowable number of transmission means that can be in range of a receiving means at once; (e) the spacing of the receiving means; (f) the acceptable rate of patient condition data loss; (g) overhead associated with transmission of the burst of patient condition data; and (h) the average repletion rate of the data bursts.

The data to be transmitted preferably includes the patient's rate and rhythm information, as well as a patient identification, the technical status of the transmitter, and error-checking information. The heart rate is a number in the range of 0 to perhaps 300, and therefore can be represented in 9 bits of information. In addition to this, a few flags or codes are desirable to indicate rhythm alarms. Therefore, the patient heart rate and rhythm information will fit in two bytes, or 16 bits. Allowing 3 bytes for the patient identification provides over 16 million unique identifications possible. The technical status needs only a few indicators such as low battery or electrode faults, so one byte is adequate. Finally, one byte can be used for error checking. Therefore, a total of 7 bytes is transmitted by transmission means 14.

A certain amount of time is required to power up and stabilize transmission means RF transmitter 20, as well as to shut it down. The above described data is framed in such a way that receiving means, R1-R4, can synchronize to the burst of data from transmission means 14 and extract individual data fields. The data framing consist of two parts. The first is the preamble, which contains some void data bytes during which time receiving means, R1-R4, stabilize and synchronize on the incoming data. This is followed by a header, which contains an unambiguous marker of the start of the data. The header function can be achieved in two bytes, and 4 bytes is a reasonable length for the preamble, although this is strongly dependent on the receiver technology adopted. Therefore, an additional 6 bytes, plus the power up and power down time of transmitter 20 are required.

Based upon the above, it is preferred that 13 bytes be transmitted by transmission means 14. Although each byte contains 8 bits of data, it actually requires 10 bits to transmit in asynchronous format. The time this takes depends on the data rate, and thus, on the transmission means transmitter 20. One available commercial miniature transmitter module has a maximum data rate of 115,200 bits per second, and could send this data in 1.13 ms. However, higher link reliability in the face of noise and interference can be achieved by using less than the maximum data rate. Therefore, as an example, if the data is sent at one quarter of this rate, 4.51 ms is required. This same transmitter module takes less than 50 µs each to power up and down, making the total transmitter "on" time 4.61 ms.

An interval of one update per second appears more than adequate. For example, most bedside monitors only update their numeric displays at 2-second intervals. Therefore, the transmitter would operate at a duty cycle of 4.61 ms out of every second, or about 0.5%. This duty cycle is low enough that the other aspects of the compromise, relating to interference, discussed above are not difficult to maintain. Further, this very low duty cycle is promising from the standpoint of battery life, since it means that very little average power is required for the transmission means transmitter 20. If more data were to be sent in each burst, such as a rudimentary waveform, the duty cycle would become greatly increased. For example, if an ECG waveform sampled at 100 points/second (corresponding to a poor recording) were to be continuously sent, the duty cycle would increase to almost 4%. This would greatly increase the probability of interference between transmitters 20, and lost data. However, because the waveform is not as redundant as the simple numerical data, the impact of occasional lost data is much greater. Given the problems with transmitting full waveforms, representative samples of waveform may be transmitted at certain times. In particular, such a sample can be taken at the time an alarm condition is detected.

Figure 2 illustrates a perspective view of the preferred embodiment of the patient transmission means 14 which comprises a subject portion 24 and a transmitter portion 26, both of which are circumscribed by broken line boxes.

Subject portion 24 of transmission means 14 comprises signal sensing means, such as ECG electrodes 16, a power supply 28, patient identifier means 30 and a support 32 (Figure 2), which is preferably adhesive and disposable.

Power supply 28 may comprise a battery, for example, lithium coin cells. These cells take the form of a flat disk, similar in size to a stack of one or two quarters. Patient identifier means 30 may comprise a device containing a numerical identifier, or serial number, such as a memory device, for example, a serial PROM. Since power supply 28 is part of subject portion 24, a fresh power supply 28, preferably a battery, is automatically provided for each patient. The device is activated when transmitter portion 26 is attached to subject portion 24.

Figure 3 is a block diagram of transmission means 14 showing the internal parts of transmitter portion 26 and subject portion 24.

Transmitter portion 26 is removably connected to subject portion 24. The patient's ECG is picked up by electrodes 16, passed through an amplifier 34 and into a computing means 18 and an analog-to-digital converter 36. Computing means 18 may comprise a microprocessor, microcontroller, ASIC, or other programmable logic. A number of electrical contacts 40 are provided as part of subject portion 24 so that the reusable transmitter portion 26 can be attached, mechanically as well as electrically. Patient identifier means 30 is also connected to computing means 18. Computing means 18 performs an analysis and outputs patient condition data to RF transmitter 20 which has an associated antenna 42 for broadcasting the patient condition data, according to the telemetry scheme outlined above.

ECG electrodes 16 are just a few inches apart on support 32. While this does not provide a conventional ECG vector (for example, lead II), it does provide a signal that is useful for basic rate and rhythm measurement. However, should this signal be inadequate, there are modifications to the structure that will allow a conventional electrode placement to be used. For example, the second electrode could be located remotely and connected by a wire to support 32. However, the use of closely spaced electrodes on a single support provides a desirable simplicity and a clean design.

Alternatively, subject portion 24 may be configured as a belt wrapped around the chest. In this case, wider electrode spacing, approximating a conventional Lead I ECG, is possible.

Patient identifier means 30 may comprise a tiny inexpensive integrated circuit encoding a unique serial number and patient ID for each support. Such devices are available commercially with unique serial numbers already installed by the manufacturer, such as the "Silicon Serial Number" made by Dallas Semiconductor (Dallas, Texas). When the adhesive support 32 is manufactured, the imbedded serial number integrated circuit is interrogated, and a matching number is printed on bar code label 33 attached to support 32 to facilitate patient admission.

Off-the-shelf technology is available for RF transmitter 20. For example, RF Monolithics (Dallas, Texas) manufactures very small RF transmitters that require only a few tiny support components. Their TX6000 series measures nominally 7mm by 10mm by 2 mm, and incorporates the entire RF function except for the antenna. Other manufacturers offer similar products.

As indicated above, a microcontroller chip may be used for transmitter portion computing means 18. These chips often include an internal analog to digital converter of suitable quality for acquiring the ECG signal. The chip should be computationally powerful enough to analyze the rhythm of the acquired patient ECG signal. While rhythm analysis is a complex subject, in this case the primary goal is to reliably identify lethal arrhythmias, in particular, those rhythms that would be considered "shockable" by an automatic external defibrillator (AED). Poor specificity between different types of lethal arrhythmias is not of great concern, since the response to any such arrhythmia is likely to be the same, the dispatch of an intervention team. Algorithms far simpler than those used for complete rhythm analysis can be used to identify shockable rhythms, such that satisfactory rhythm identification can be performed with a fairly simple and low power microprocessor. In general, such simplified algorithms concentrate on the timing of the heart beats, rather than the details of their morphology. For example, the arrhythmias of ventricular tachycardia and ventricular fibrillation may be identified on the basis of high apparent heart rate, without making an effort to distinguish between them on the basis of waveform shape, as in both cases an alarm condition would be declared. Potential devices include various members of the PIC family made by Microchip (Chandler, Arizona), AVR devices made by Atmel (San Jose, California), and 430 series microcontrollers by Texas Instruments (Dallas, Texas).

Amplifier 34 used to receive the signal from electrodes 16 is much simpler than the circuits found in conventional monitors. Because the device is body-worn and has no interconnecting lead wires or cables, the 60Hz common mode rejection problems that challenge conventional monitors are nonexistent. Much of the dynamic range of conventional ECG circuits is occupied by the need to accept, and later filter out, low frequency phenomenon, such as the DC offset voltage present at the electrodes and baseline wander. However, the ECG signal in the present system 10 is used primarily for rate and rhythm analysis. The first operation performed in such analysis is often to severely high pass filter the ECG signal. For example, the rate-meter in many monitors utilizes an approximately 10 Hz high pass filter. If the amplifier is coupled to the ECG electrodes through capacitors, rather than directly, this high pass filtering can be performed before the signal even enters the amplifier. In this way, none of the dynamic range of the amplifier is wasted on DC offsets and other low-frequency artifacts. Therefore, a very modest circuit is used for amplifier 34. Further, this arrangement relieves the computing means 18 of the need to perform such filtering in software. Such a simplified amplifier can be constructed very compactly, using the highly miniaturized components available today. However, consideration is given to compatibility with patients having implanted pacemakers. Additional electronics and signal processing is required to identify and reject the pacemaker spikes, so that they do not interfere with the beat triggering.

In addition to the circuits shown on the block diagram, it is necessary to perform self-testing. In particular, the battery level must be monitored, the quality of the electrode contact verified, and all of the internal signal acquisition and processing functions checked. Analog to digital converter 36 embedded in computing means 18 has an input multiplexer (not shown) that allows it to also check the battery voltage. Computing means 18 can inject test currents into electrodes 16 to verify their impedance. Similarly, a test pulse can be injected into amplifier 34 to verify its gain. Various software checks can be used to verify the internal operation of computing means 18.

The average current consumption of each component is the product of its operating current and duty cycle. The following estimates assume a 3 volt lithium battery as the power source. RF transmitter 20 consumes 5µA on standby, and 12mA when active. The average active current is therefore 12mA times the 0.5% active duty cycle, or 60µA. The average standby current is then 5µA times the 99.5% standby duty cycle, or nearly 5µA. Amplifier 34 operates on a 100% duty cycle, with 250µA of current. Computing means 18, including internal analog to digital converter 36, consumes an average current of 600µA. Patient identifier means 30 is disabled after it is initially interrogated, and therefore contributes nil to the average current consumption. The total average current consumption is the sum of these average figures, or 915µA.

The power consumption estimate can be evaluated with respect to the capacities of some typical batteries. Inexpensive lithium coin cells are available with diameters in the range of 20 to 23 mm, and thickness varying from 1.6 to 3.2 mm, according to capacity. All of these cells cost under one dollar in quantity, with the least expensive being under 30 cents. Capacities range from 100 to 255 mA hours. Since the estimated current of the device is just under 1 mA, run times of 100 to 250 hours, or 4 to 10 days, are achievable with these inexpensive batteries.

The range of patient transmission means 14 is on the order of the size of a patient room or ward. Due to the short range of patient transmission means 14, receiving means must be placed at frequent intervals, such as in each room. The function of the receiving means, R1-R4, is to collect the patient condition data from any patient transmission means 14 within its range. This data is then merged with an identifier of the receiving means location, and transmitted to central station means 12.

The manufacturers of RF transmitter 20 also produce complementary receiver modules, which may be used in the receiving means. However, in the interests of achieving higher performance, it is desirable to use a somewhat more sophisticated receiver. In particular, it is desirable that the receiver comprise a means for quantify the signal strength of the received patient condition data, as this is helpful in refining patient location when patient condition data is being received by more than one receiving means. In addition to considering signal strength, the phase or time of arrival of the received patient condition data at multiple receiving means can be used to refine the estimate of the transmission means location, by well-known triangulation means. Accordingly, it is preferred that the receiving means, R1-R4, comprise a means for keeping track of the phase and/or time of arrival of the received patient condition data.

Once the patient condition data has been received and merged, it must be communicated to central station means 12. Since many receiving means may be connected to a single central station means 12, some type of networked link to central station means 12 is desirable. This could be a wired connection, such as Ethernet. However, due to the large number of receiving means in some installations, the use of wired connections may be costly, due to the expense of installation of the wiring. In these cases, a second wireless link, from the receiving means to the central station means, is preferable. Wireless computer networking products are commercially available and therefore satisfactory technology is available off the shelf. These devices typically duplicate the functionality of wired Ethernet via their wireless links. An example is a 2.4 GHz network operating with IEEE 802.11 protocol, available as standard product from several manufacturers. Since the receiving means can be operated from the AC line, there are no special constraints regarding power consumption.

The wireless network products are available as small modules or cards that can be embedded into a product. In addition to means for communicating with central station means 12, the receiving means R1-R4 may optionally also contain a computing means, such as a microprocessor, which performs error checking of the received data, appends the identifier of the receiving means location and received signal strength indicator, and controls the communication of this merged data over the networked link to the central station. However, this too is a physically small device, allowing the entire receiving means to be made in a small enclosure self-supported by prongs that fit into an AC outlet, similar to common power adapter units. Therefore, installation is as simple as plugging the receiving means into an outlet in each room.

Central station means 12 receives the patient condition data from receiving means R1-R4, either by wire or wireless network. Central station means 12 may consist of custom software running on a PC, equipped with suitable commercial wired or wireless network connectivity. Central station means 12 performs a first task of sorting the received patient condition data, as data may have been acquired by more than one receiving means. The incoming data is then analyzed in two ways. The content of the data is analyzed for alarm conditions. High and low rate alarms could be provided at the central station means, although the detection of fatal arrhythmias is preferably performed in the transmission means. Second, the location of the receiving means receiving the strongest patient condition data signal strength is noted, providing the patient locator function. Associated with this is an evaluation of the patient condition data signal quality and monitoring of technical alarms, such as low battery, bad electrode, etc. Central station 12 means also contains the database that associates each patient's name with the numerical identifier obtained from the support serial number 33.

If desired, central station means 12 may further comprise a display for displaying the real time data for all patients, and even log this to a patient trend database. Obviously, it is possible to sound a local alarm, and indicate the patient alarm condition and location on the unit's display. However, the system may be more valuable if it also directly notifies a response team. This can be done by an interface to a paging system 44 (Figure 1). In the case of a paging system with alphanumeric capability, patient location can be transmitted. However, an interface to a voice pager or telephone system 46 (Figure 1) is also possible using speech synthesis or voice messaging technology.

Note again that the present invention is not limited to use in monitoring patients in a hospital setting. Rather the invention may be used to monitor conditions of any subject, including animate and inanimate objects, in a defined area. When monitoring the cardiac health of patients, the preferred embodiment uses an ECG waveform as a subject or patient signal; however, other signals indicative of the heart rate and rhythm can be used. The ECG is a convenient signal that may be acquired with high reliability and a minimum expenditure of power. Similar information, however, can be obtained from a plethysmographic signal. For example, a photoplethysmograph sensor could be arranged to operate in the reflectance mode, such that a plethysmographic signal is obtained from the tissue beneath the device. This signal would take the place of the ECG for rate and rhythm monitoring. In this case, the device need not be applied over the chest; it could be attached to any suitably perfused tissue. Alternatively, the device could be configured to use a more conventional transmission mode photoplethysmogrpahic sensor, which could be applied to a suitable appendage such as the earlobe or finger. Similarly, the plethysmographic signal could be obtained by known impedance methods, such as by measuring the impedance of the tissue beneath the device by means of suitable electrodes.

Thus, it is understood, that while particular examples have been described it should be apparent to those skilled in the art that many modifications can be made without departing from the scope and intent of the invention. Accordingly, the invention is not limited to the specific embodiments thereof except as defined in the appended claims.

## Claims

1. A monitoring system (10) for monitoring each of a plurality of patients, comprising:
one or more transmitters (14), each associated with a respective patient and that acquires, analyses a subject signal for subject condition and transmits a patient signal carrying physiological patient data resulting from the analysis for the respective patient;
one or more receivers (R1-R4) that are configured to receive the patient signals from the transmitters, to generate corresponding receiver signals that include the physiological patient data and to communicate the receiver signals to a central station (12), said central station (12) receiving and analyzing the physiological patient data;
**characterized in that**
communication between the transmitters (14) and receivers (R1-R4) is unidirectional, from the transmitters (14) to the receivers (R1-R4); and
at least two of the transmitters (14) transmit on an identical transmission channel and each transmission from the transmitters (14) comprises asynchronous cycles, each cycle having an active phase in which energy modulated with the data is emitted and a longer inactive phase in which no energy is emitted, the duration of the active phase being less than the period of a complete cycle divided by a predetermined maximum number of transmitters (14); and
the transmitters (14) transmit their respective patient signals asynchronously and uncoordinated, such that overlap on the identical transmission channel is possible; and
each receiver (R1-R4) has a coverage area (22) in which it is capable of receiving patient signals from transmitters located within the respective coverage area (22), the coverage area (22) of each receiver overlapping that of immediately adjacent receivers.

2. The monitoring system as claimed in claim 1 wherein the central station (12) alerts a user when the physiological patient data indicates a predetermined alert condition.

3. The monitoring system as claimed in claim 1 wherein the period of the cycles is variable.

4. The monitoring system as claimed in claim 1 in which the physiological patient data includes patient heart rate and rhythm data.

5. The monitoring system as claimed in claim 1 in which the receivers are also configured to transmit location data to the central station (12).

6. The monitoring system as claimed in claim 5, in which the location data includes receiver identification information, such that the central station determines the location of each patient according to from which receiver(s) the central station receives the respective patient signal and location data..

7. The monitoring system as claimed in claim 6, wherein:
each receiver (R1-R4) includes means for measuring signal strength of the patient signal received from the respective transmitter;
the location data further includes an indication of signal strength,
the central station (12) locates each patient as a function of the signal strength indications and the receiver identification information of the receivers that received the patient signal for the respective patient.

8. The monitoring system as claimed in claim 6, wherein
each receiver (R1-R4) comprises means for measuring the time of arrival of the patient signals received from the transmitters;
the location data includes time-of-arrival data; and
the central station (12) locates each patient as a function of the time-of-arrival data and the receiver identification information of the receivers that received the patient signal for the respective patient.

9. The monitoring system as claimed in claim 1 wherein the central station (12) further comprises an interface to a phone or paging system.

## Patentansprüche

1. Überwachungssystem (10) zum Überwachen eines jeden von mehreren Patienten, das Folgendes umfasst:
einen oder mehrere Sender (14), die jeder einem jeweiligen Patienten zugeordnet sind und ein Personensignal erfassen, auf einen Personenzustand hin analysieren und ein Patientensignal übertragen, das physiologische Patientendaten, die sich aus der Analyse ergeben, für den jeweiligen Patienten befördert,
einen oder mehrere Empfänger (R1-R4), die dafür konfiguriert sind, die Patientensignale von den Sendern zu empfangen, entsprechende Empfängersignale zu erzeugen, welche die physiologischen Patientendaten einschließen, und die Empfängersignale zu einer Zentralstation (12) zu übertragen, wobei die Zentralstation (12) die physiologischen Patientendaten empfängt und analysiert,
**dadurch gekennzeichnet, dass**
die Kommunikation zwischen den Sendern (14) und den Empfängern (R1-R4) einseitig, von den Sendern (14) zu den Empfängern (R1-R4), gerichtet ist und
wenigstens zwei der Sender (14) auf einem identischen Übertragungskanal senden und jede Übertragung von den Sendern (14) asynchrone Zyklen umfasst, wobei jeder Zyklus eine aktive Phase, in der mit den Daten modulierte Energie emittiert wird, und eine längere inaktive Phase, in der keine Energie emittiert wird, hat, wobei die Dauer der aktiven Phase geringer ist als die Zeitdauer eines vollständigen Zyklus, dividiert durch eine vorbestimmte maximale Zahl von Sendern (14), und
die Sender (14) ihre jeweiligen Patientensignale asynchron und unkoordiniert übertragen, so dass eine Überlappung auf dem identischen Übertragungskanal möglich ist, und
jeder Empfänger (R1-R4) ein Abdeckungsgebiet (22) hat, in dem er dazu in der Lage ist, Patientensignale von Sendern zu empfangen, die sich innerhalb des jeweiligen Abdeckungsgebiets (22) befinden, wobei das Abdeckungsgebiet (22) jedes Empfängers dasjenige der unmittelbar benachbarten Empfänger überlappt.

2. Überwachungssystem nach Anspruch 1, wobei die Zentralstation (12) einen Benutzer warnt, wenn die physiologischen Patientendaten einen vorbestimmten Alarmzustand anzeigen.

3. Überwachungssystem nach Anspruch 1, wobei die Zeitdauer der Zyklen veränderlich ist.

4. Überwachungssystem nach Anspruch 1, wobei die physiologischen Patientendaten Herzfrequenz- und -rhythmusdaten einschließen.

5. Überwachungssystem nach Anspruch 1, wobei die Empfänger ebenfalls dafür konfiguriert sind, Positionsdaten an die Zentralstation (12) zu übertragen.

6. Überwachungssystem nach Anspruch 5, wobei die Positionsdaten Empfänger-Identifikationsdaten einschließen derart, dass die Zentralstation die Position jedes Patienten dementsprechend bestimmt, von welchem/n Empfänger(n) die Zentralstation das jeweilige Patientensignal und die Positionsdaten empfängt.

7. Überwachungssystem nach Anspruch 6, wobei:
jeder Empfänger (R1-R4) Mittel zum Messen der Signalstärke des von dem jeweiligen Sender empfangenen Patientensignals einschließt,
die Positionsdaten ferner eine Anzeige der Signalstärke einschließen,
die Zentralstation (12) jeden Patienten in Abhängigkeit von den Signalstärkeanzeigen und den Empfänger-Identifikationsinformationen derjenigen Empfänger lokalisiert, die das Patientensignal für den jeweiligen Patienten empfingen.

8. Überwachungssystem nach Anspruch 6, wobei:
jeder Empfänger (R1-R4) Mittel zum Messen der Ankunftszeit der von den Sendern empfangenen Patientensignale einschließt,
die Positionsdaten Ankunftszeitdaten einschließen und
die Zentralstation (12) jeden Patienten in Abhängigkeit von den Ankunftszeitdaten und den Empfänger-Identifikationsinformationen derjenigen Empfänger lokalisiert, die das Patientensignal für den jeweiligen Patienten empfingen.

9. Überwachungssystem nach Anspruch 1, wobei die Zentralstation (12) ferner eine Schnittstelle zu einem Telefon- oder Funkrufsystem umfasst.

## Revendications

1. Système de surveillance (10) pour surveiller chacun d'une pluralité de patients, comprenant :
un ou plusieurs émetteurs (14), chacun étant associé à un patient respectif et qui acquiert et analyse un signal de sujet pour une condition de sujet et
transmet un signal de patient portant des données physiologiques de patient découlant de l'analyse pour le patient respectif ;
un ou plusieurs récepteurs (R1-R4) qui sont configurés pour recevoir les signaux de patient des émetteurs, pour générer des signaux de récepteur correspondant comprenant les données physiologiques de patient et
pour communiquer les signaux de récepteur à une station centrale (12), ladite station centrale (12) recevant et analysant les données physiologiques de patient ;
**caractérisé en ce que**
la communication entre les émetteurs (14) et les récepteurs (R1-R4) est unidirectionnelle, des émetteurs (14) aux récepteurs (R1-R4) ; et
au moins deux des émetteurs (14) transmettent sur un canal de transmission identique et chaque transmission des émetteurs (14) comprend des cycles asynchrones, chaque cycle ayant une phase active dans laquelle l'énergie modulée avec les données est émise et une phase inactive plus longue dans laquelle aucune énergie n'est émise, la durée de la phase active étant inférieure à la période d'un cycle complet divisé par un nombre maximum prédéterminé d'émetteurs (14) ; et
les émetteurs (14) transmettent leurs signaux de patient respectifs de manière asynchrone et non coordonnée, de sorte qu'un chevauchement sur le canal de transmission identique soit possible ; et
chaque récepteur (R1-R4) a une zone de couverture (22) dans laquelle il est capable de recevoir des signaux de patient d'émetteurs situés dans la zone de couverture respective (22), la zone de couverture (22) de chaque récepteur chevauchant celle de récepteurs immédiatement adjacents.

2. Système de surveillance selon la revendication 1, dans lequel la station centrale (12) alerte un utilisateur lorsque les données physiologiques de patient indiquent une condition d'alerte prédéterminée.

3. Système de surveillance selon la revendication 1, dans lequel la période des cycles est variable.

4. Système de surveillance selon la revendication 1, dans lequel les données physiologiques de patient comprennent des données de pouls et de rythme cardiaque de patient.

5. Système de surveillance selon la revendication 1, dans lequel les récepteurs sont également configurés pour transmettre des données d'emplacement à la station centrale (12).

6. Système de surveillance selon la revendication 5, dans lequel les données d'emplacement comprennent des informations d'identification de récepteur, de sorte que la station centrale détermine l'emplacement de chaque patient en fonction du ou des récepteurs desquels la station centrale reçoit le signal de patient respectif et les données d'emplacement respectives.

7. Système de surveillance selon la revendication 6, dans lequel
chaque récepteur (R1-R4) comprend un moyen pour mesurer la force de signal du signal de patient reçu de l'émetteur respectif ;
les données d'emplacement comprennent en outre une indication de la force de signal ;
la station centrale (12) localise chaque patient en fonction des indications de force de signal et des informations d'identification de récepteur des récepteurs qui ont reçu le signal de patient pour le patient respectif.

8. Système de surveillance selon la revendication 6, dans lequel
chaque récepteur (R1-R4) comprend un moyen pour mesurer le temps d'arrivée des signaux de patient reçus des émetteurs ;
les données d'emplacement comprennent des données de temps d'arrivée ; et
la station centrale (12) localise chaque patient en fonction des données de temps d'arrivée et des informations d'identification de récepteur des récepteurs qui ont reçu le signal de patient pour le patient respectif.

9. Système de surveillance selon la revendication 1, dans lequel la station centrale (12) comprend en outre une interface avec un téléphone ou un système de radiomessagerie.
